# EUROPEAN PATENT SPECIFICATION

(11) **EP 1 427 357 B1**
(45) Date of publication and mention of the grant of the patent: **26.03.2014**
(21) Application number: 02719525.4
(22) Date of filing: 19.04.2002
(51) Int. Cl.: A61F 2/40

(54) **MODULAR HUMERAL HEAD WITH ECCENTRIC CONNECTOR**
MODULARER OBERARMKOPF MIT EXZENTRISCHEM VERBINDER
TETE D'HUMERUS MODULAIRE A CONNECTEUR EXCENTRIQUE

(30) Priority: 19.04.2001 US 838698
(43) Date of publication of application: 16.06.2004
(73) Proprietor: Wright Medical Technology, Inc., Arlington Tennessee 38002-9501 (US)
(72) Inventor: GRUSIN, N., Kelly, Memphis, TN 38119 (US); JOBE, Christopher, Redlands, CA 92373 (US); BROOKS, Michael, L., Memphis, TN 38128 (US); RIVAS, Felix, Jr., Cordova, TN 38016 (US); DUKE, Edward, Bartlett, TN 38134 (US)
(74) Representative: Zounek, Nikolai
(86) International application number: PCT/US2002/012440
(87) International publication number: WO 2002/085259

(56) References cited:
- US-A- 5 358 526
- US-A- 5 507 818
- US-B1- 6 197 062

## Description

### Field of the Invention

The present invention relates to shoulder prostheses. More particularly, the present invention relates to a modular humeral head with eccentric connector that allows the modular humeral head to be connected to a humeral stem with varying degrees of eccentricity.

### Background of the Invention

During the procedure of a shoulder replacement operation, at least a portion of the proximal section of the humeral shaft will be replaced by a prosthesis. Early shoulder prostheses attempted to directly replicate the upper portion of the humerus which they were designed to replace. Thus, like the natural upper portion of the humerus, they were typically unitary structures including a stem to be implanted within the humerus and a head to be positioned within the glenoid cavity of the scapula.

There are several problems with the a conventional unitary shoulder prosthesis. In order to accommodate the varying morphologies of patients, it is necessary to maintain a large inventory of differently configured prostheses. Not only are prostheses with different sizes of heads and stems required but also prostheses with the head and stem configured with varying radial offsets relative to one another. These various configurations are required in each size category.

To reduce the required inventory, assorted modular prostheses have been devised. A modular prosthesis generally consists of two parts: a stem that is mounted into the medullary canal of the humerus, and a head component connected in some manner to the stem. The head component replaces the bearing surface of the humeral head to allow the movement of the shoulder. Different stem sizes and head sizes in a modular prosthesis provide the surgeon with some degree of interoperative flexibility which facilitates reconstruction of the original anatomy of the patient. With a range of stem sizes and a range of head sizes available, the surgeon can choose a particular combination to suit the anatomy of each individual without having to have a large inventory of unitary humeral prostheses.

However, individual patients may also require differing offsets between the axis of the head and the axis of the stem. For example, in one patient the posterior offset may be greater than in another patient. To meet this need, some prior art prostheses provide a plurality of connecting members for connecting the head to the stem with different offsets.

U.S. Patent 5,358,526 discloses a modular humeral prosthesis comprising a humeral stem, a humeral head and a wedge-shaped spacer for connecting the head to the stem. The wedge-shaped spacer includes a first face which fits a bearing face on the stem and a second face for attaching the head. The head has a semi-spherical cap and a base with a conical bore. The bore is positioned off-center on the base i.e. non-concentrically relative to the head. A conical stud protruding from the second face of the wedge-shaped spacer is designed to fit into the bore.

Differently configured connecting members are designed to provide varying degrees of eccentricity, or offset. Thus, although prior art modular prostheses have reduced the need of an inventory of differently configured unitary prostheses, an inventory of differently configured connecting members for connecting the humeral head to the stem is still required.

Accordingly, there is a need for a modular shoulder prosthesis which allows eccentricity adjustment without requiring an inventory of connecting members.

### Summary of the Invention

The present invention relates to shoulder prostheses in accordance with the features of claim 1 in which a humeral head, chosen to suit a patient, is attached to a stem' chosen to suit the resected humerus of the patient, by means of an intermediate connecting member. The intermediate connecting member can be adjusted to vary the eccentricity of the humeral head to a portion of the connecting member attachable to the stem. Two degrees of eccentricity, preferably, are provided.

The modular humeral prosthesis generally comprises a stem to be fitted to a resected humerus, a head sized and configured to approximate the humeral head, and an intermediate connecting member for connecting the stem to the head. A locking piece is provided to secure the intermediate connecting member to the head. The humeral head has a spherical surface formed about an axis on one side and a flat face on the opposite side. A first degree of eccentricity is provided by an opening positioned eccentrically (i.e., not centered) on the flat face of the head for receiving the connecting member. The opening is preferably a threaded, shallow bore.

The connecting member is formed of a first portion for connection with the head and a second portion for connection with the stem. A second degree of eccentricity is provided by the relationship of the second portion to the first portion. The first portion, for example, may be a shallow, round plate. The second portion, for example, a male connecting piece of substantially smaller diameter than the plate, is located non-coaxially on the plate and extends generally conically therefrom.

Preferably, a locking piece is used to secure the connecting member in place in the humeral head. The locking piece, preferably, is a threaded ring that is inserted over the plate of the connecting member and threaded through the bore in the head. In securing the connecting piece to connect the head to the stem, the plate is loosely inserted into the shallow bore on the humeral head. The connecting member is rotated until the desired eccentricity of the male connecting piece to the humeral head is achieved. The locking piece is inserted and threaded in the shallow bore on the humeral head to lock the connecting member in place in the humeral head. The male connecting piece fits into the conical cavity of the stem for, e.g., a Morse taper fit.

Thus, a single connecting member may provide a great variation of eccentricity of the humeral head to a portion of the connecting member attachable to the stem. This is especially advantageous in that it allows a surgeon to customize the eccentricity of the humeral head to a patient's specific morphology without requiring an extensive inventory of modular components.

### Brief Description of the Drawings

Figure 1 a cross-section view of one embodiment of the present invention;
Figure 2 is a bottom view of one embodiment of the humeral head of the present invention;
Figure 3 is a cross-sectional view of one embodiment of the humeral head of the invention;
Figure 4 is a bottom view of one embodiment of the connecting member of the present invention;
Figure 5 is a side view of one embodiment of the connecting member of the present invention;
Figure 6 is a side view of one embodiment of the locking piece of the present invention;
Figure 7 is a top view of one embodiment of the locking piece of the present invention;
Figure 8 is a bottom view of one embodiment of the invention.

### Detailed Description of the Preferred Embodiments

In the drawing, like structures are provided with like reference numerals.

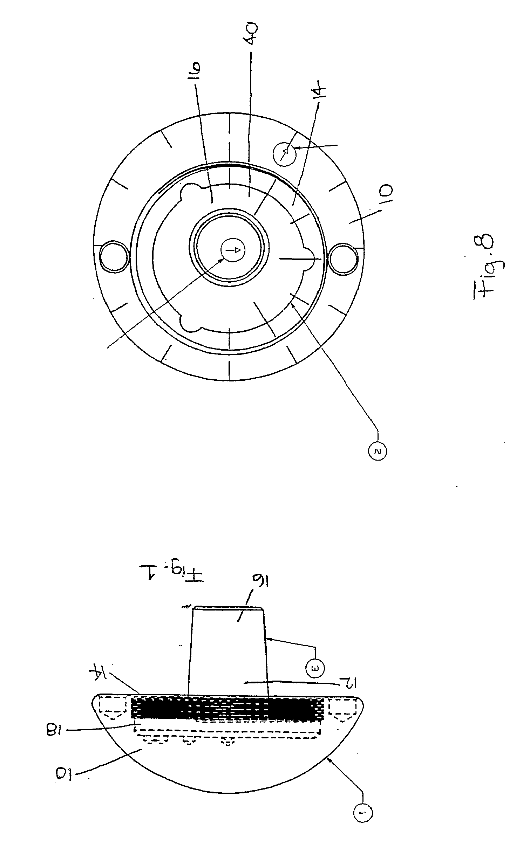

Figure 1 illustrates a cross-sectional view of a first embodiment of the present invention. A shallow bore, generally 18, is positioned non-coaxially on a flat face of humeral head 10. In one embodiment, humeral head 10 locks to a connecting member 12 by a locking piece 14. Connecting member 12 includes a first portion 15 for connection with the head and second portion 16 for connection with the stem. The first portion 15 may be a shallow, generally flat, round plate. Second portion 16 is a male connecting piece, of substantially smaller diameter than the first portion, located eccentrically on the first portion and extending generally conically therefrom. Second portion 16 fits into a conical cavity of the stem for a Morse taper fit. Locking piece 14 locks the connecting member in place in the humeral head. Preferably, locking piece 14 is a threaded ring that is inserted over the first portion of connecting member 12 and threaded through bore 18 in the head. A peripheral shoulder of the locking piece is provided to engage the first portion of the connecting member.

To secure connecting member 12 to humeral head 10, the flat round plate is loosely fit into shallow bore 18 of humeral head 10. A first degree of eccentricity is achieved by rotating connecting member 12 until the desired offset of second portion 16 to humeral head 10 is achieved. To secure connecting member 12 in place in humeral head 10, locking piece 14 is inserted and threaded in shallow bore 18 on humeral head.

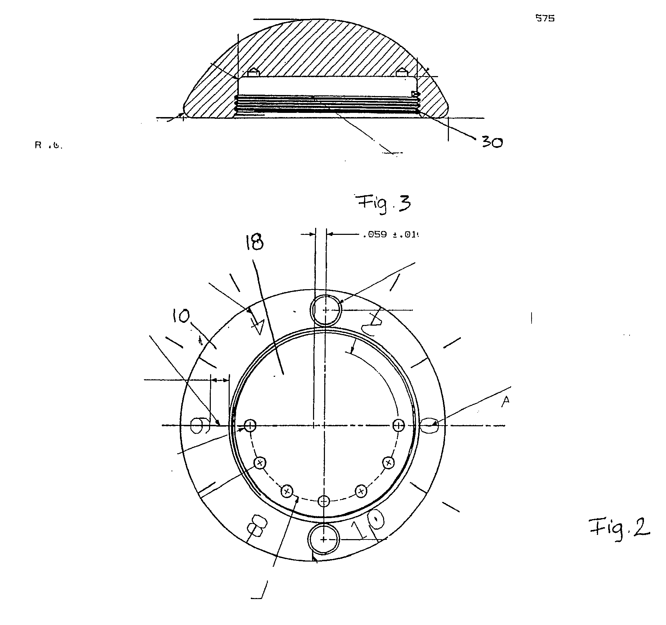

As seen in Figure 2, bore 18 is located eccentrically on the flat bottom surface of humeral head 10; that is, the axes of the spherical head and the bore are spaced apart. Bore 18 is of relatively large diameter when compared to the second portion of the connecting member. To receive the first portion of the connecting member, bore 18 is relatively shallow. Optionally, bore 18 is threaded to engage with the locking piece to secure the connecting member in place in humeral head 10 at a desired degree of eccentricity.

Figure 3 shows a cross-section view of the humeral head and depicts how bore 18 may be eccentrically located in humeral head 10. Threads 30 line bore 18 for engaging with the locking member.

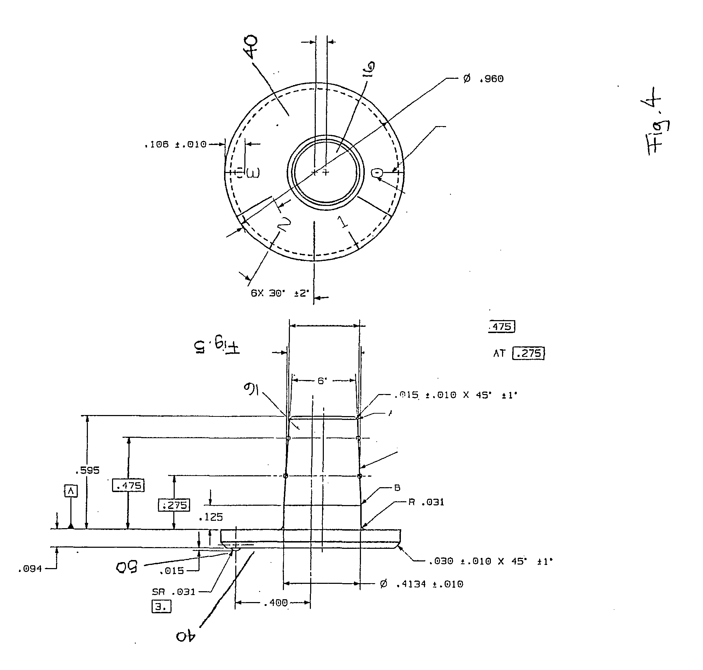

Figure 5 illustrates a side view of connecting member 12. As is shown in Figure 5, first portion 15 is relatively shallow while second portion 16 is relatively deep. Thus, first portion 15 is configured to fit into the shallow bore of the humeral head. Second portion 16 is preferably configured for a Morse taper fit with the humeral stem. However, any configuration of the second portion that allows the second portion to engage the humeral stem may be used. For example, second portion could be of a dove-tail slot configuration To provide adjustable positioning of connecting member 12 within the bore while preventing undesirable movement, detent 50 is preferably provided in addition to the locking piece. A series of receiving cavities are provided in the bore of the humeral head for engaging detent 50. Thus, connecting member 12 may be rotatably adjusted in the bore into predetermined positions.

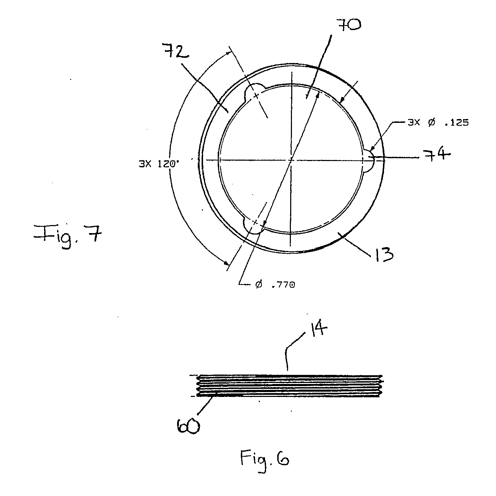

As seen in Figure 6, locking piece 14 is of a depth such that both the first portion of the connecting member and locking piece 14 may be inserted into the shallow bore of the humeral head. The outer circumference of locking piece 14 has threads 60 for threadably engaging the bore of the humeral head. Locking piece secures the connecting member in place within the humeral head at a desired eccentricity of the second portion (for engaging the humeral stem) to the humeral head is achieved.

Figure 7 provides a top view of locking piece 14. Preferably, locking piece 14 is generally configured as a ring. However, any configuration suitable for securing the connecting member in place in the humeral head may be used. Along the inside circumference of locking piece 14 are notches 74 for engaging a tool for rotating the locking piece.

As seen in Figure 8, when the components of the invention are fit together, the second portion for engaging the humeral stem may be adjusted to be positioned centrally or eccentrically on the humeral head. Shallow bore 18 is eccentrically located on the flat face of humeral head 10 and receives first portion 15 of connecting member 12, which is generally shallow and round. Second portion 16, in this embodiment, is a generally conical piece located eccentrically upon and extending conically from first plate-like portion 15. By rotating connecting member 12 within bore 18, second portion 16 may be adjusted to be located at a desired eccentricity to humeral head 10. Preferably, locking piece 14 is fit over first portion 15 of connecting member 12 and threaded into bore 18. A tool may engage notches 74 for adjusting locking piece 14.

Thus, as is described above, the eccentricity the humeral head to a portion of the connecting member attachable to the stem may be adjusted in two different ways as provided by the connecting member. First, the bore within the humeral head is non-coaxial with the humeral head. Thus, in selecting the placement of the connecting member within the bore, a first degree of eccentricity is achieved. Second, the second portion of the connecting member is non-coaxial with the first portion of the connecting member. Thus, by rotating the connecting member within the bore, a second degree of eccentricity is achieved.

While various embodiments in accordance with the present invention have been shown and described, it is understood that the invention is not limited thereto, and is susceptible to numerous changes and modifications as known to those skilled in the art. Therefore, this invention is not limited to the details shown and described herein, and includes all such changes and modifications as encompassed by the scope of the appended claims.

## Claims

1. A modular shoulder prosthesis for replacement of the humeral head of a humerus, comprising:
a stem having a distal end for insertion in the superior end of the medullary cavity of the humerus and a proximal end for engagement with a humeral head (10);
a head (10) for positioning in a glenoid cavity, the head (10) having, on one side, a generally spherical surface (1) formed about an axis and, on the other side, a generally flat face bounding the spherical surface;
a bore (18) being formed in the flat head face, the bore (18) including an axis which is non-coaxial with the axis of the generally spherical surface of the head (10);
a connecting member (12) for connecting the head (10) and stem, the connecting member (12) having a first portion (15) and a second portion (16), the first portion (15) configured for insertion into the bore (18) of the head (10), the bore (18) being positioned
eccentrically, i.e., not centered, on the flat face of the head (10) for receiving the connecting member (12) and providing a first degree of eccentricity; a locking piece (14) for locking the connecting member (12) non-rotatably within the bore (18); wherein
the second portion (16) is a male connecting piece, of substantially smaller diameter than the first portion (15), extending conically from the first portion (15) and fits into a conical cavity of the stem for a Morse taper fit,**characterised in that** the second position (16) extends axially parallel offset from the first portion (15) to provide a second degree of eccentricity by the relationship of the second portion (16) to the first portion (15).

2. The prosthesis of claim 1 wherein the first portion (15) of the connecting member (12) is configured for rotatable insertion in the axial bore (18) of the humeral head (10) to enable adjustment of the second portion (16) of the connecting member (12) with respect to the axis of the spherical portion.

3. The prosthesis of claim 1 wherein the second portion (16) of the connecting member (12) is a protruding pin having a Morse taper.

4. The prosthesis of claim 1 wherein the bore of the humeral head (10) and the locking piece (14) are threaded for engagement.

5. The prosthesis of claim 1 wherein the locking piece (14) is a locking ring having a bore therethrough with an inner surface and a peripheral shoulder engaging the first portion (15) of the connecting member (12).

6. The prosthesis of claim 5 wherein at least one groove is provided along the inner surface of the ring for receiving a tool for adjusting the ring.

7. The prosthesis of claim 1 wherein the locking piece (14) includes a tool engaging surface for receiving a tool for adjusting the locking piece (14).

8. The prosthesis of claim 1 further comprising a detent formed on the first portion (15) of the connecting member (12) and a series of recesses formed within the axial bore (18) of the humeral head (10) for engaging the detent to enable the connecting member (12) to be rotatably adjusted with respect to the humeral head (10) into predetermined positions.

## Patentansprüche

1. Modulare Schulterprothese zum Ersetzen des Humeruskopfes eines Humerus, umfassend:
einen Schaft mit einem distalen Ende zum Einsetzen in das obere Ende der Markhöhle des Humerus und ein proximales Ende zum Eingreifen in einen Humeruskopf (10);
einen Kopf (10) zum Positionieren in einer Glenoidhöhle, wobei der Kopf (10) auf einer Seite eine allgemein kugelförmige, um eine Achse gebildete Oberfläche (1) aufweist und auf der anderen Seite eine allgemein flache, an die kugelförmige Oberfläche angrenzende Fläche aufweist;
eine Bohrung (18), die in der flachen Kopffläche gebildet ist, wobei die Bohrung (18) eine Achse aufweist, die nicht koaxial zu der Achse der im Allgemeinen kugelförmigen Oberfläche des Kopfes (10) ist;
ein Verbindungselement (12) zum Verbinden von Kopf (10) und Schaft, wobei das Verbindungselement (12) ein erstes Teil (15) und ein zweites Teil (15) aufweist, konfiguriert zum Einsetzen in die Bohrung (18) des Kopfes (10), wobei die Bohrung (18) exzentrisch, d. h. nicht zentriert, auf der flachen Seite des Kopfes (10) positioniert ist, um das Verbindungselement (12) aufzunehmen und um einen ersten Grad an Exzentrizität bereitzustellen;
ein Verriegelungsstück (14), um das Verbindungselement (12) nicht drehend in der Bohrung (18) zu verriegeln; wobei
das zweite Teil (16) ein Steckerteil von einem im Wesentlichen kleineren Durchmesser als das erste Teil (15) ist, konisch verlaufend von dem ersten Teil (15) und passend in den konischen Hohlraum des Schafts für einen Morsekegel, **dadurch gekennzeichnet, dass** das zweite Teil (16) axial parallel versetzt von dem ersten Teil (15) verläuft, um einen zweiten Grad an Exzentrizität durch die Beziehung des zweiten Teils (16) zum ersten Teil (15) bereitzustellen.

2. Prothese nach Anspruch 1, wobei das erste Teil (15) des Verbindungselements (12) zum drehbaren Einsetzen in die Axialbohrung (18) des Humeruskopfes (10) konfiguriert ist, um die Anpassung des zweiten Teils (16) des Verbindungselements (12) in Bezug auf die Achse des kugelförmigen Teils zu ermöglichen.

3. Prothese nach Anspruch 1, wobei das zweite Teil (16) des Verbindungselements (12) ein vorspringender Stift mit einem Morsekegel ist.

4. Prothese nach Anspruch 1, wobei die Bohrung des Humeruskopfes (10) und das Verriegelungselement (14) mit einem Gewinde zum Eingreifen versehen sind.

5. Prothese nach Anspruch 1, wobei das Verriegelungselement (14) ein Verriegelungsring ist, durch den eine Bohrung mit einer Innenoberfläche führt und der eine in das erste Teil (15) des Verbindungselements (12) eingreifende Umfangschulter aufweist.

6. Prothese nach Anspruch 5, wobei mindestens eine Rille entlang der Innenoberfläche des Rings zum Aufnehmen eines Werkzeuges zum Anpassen des Rings bereitgestellt ist.

7. Prothese nach Anspruch 1, wobei das Verriegelungselement (14) eine Werkzeugeingriffsoberfläche zum Aufnehmen eines Werkzeugs zum Anpassen des Verriegelungselements (14) umfasst.

8. Prothese nach Anspruch 1, ferner umfassend eine auf dem ersten Teil (15) des Verbindungselements (12) gebildete Arretierung und eine Reihe von Vertiefungen, gebildet in der Axialbohrung (18) des Humeruskopfes (10) zum Eingreifen mit der Arretierung, um die drehbare Anpassung des Verbindungselements (12) in Bezug auf den Humeruskopf in vorbestimmte Positionen zu ermöglichen.

## Revendications

1. Prothèse d'épaule modulaire destinée au remplacement de la tête humérale d'un humérus, comprenant :
une tige ayant une extrémité distale destinée à être insérée dans l'extrémité supérieure de la cavité médullaire de l'humérus et une extrémité proximale destinée à entrer en prise avec une tête humérale (10) ;
une tête (10) destinée à être positionnée dans une cavité glénoïde, la tête (10) ayant, d'un côté, une surface (1) généralement sphérique formée autour d'un axe et, de l'autre côté, une face généralement plate délimitant la surface sphérique ;
un alésage (18) étant formé dans la face de tête plate, l'alésage (18) comportant un axe qui est non coaxial avec l'axe de la surface généralement sphérique de la tête (10) ;
un organe de raccordement (12) destiné à raccorder la tête (10) et la tige, l'organe de raccordement (12) ayant une première partie (15) et une seconde partie (16), la première partie (15) étant configurée de manière à être insérée dans l'alésage (18) de la tête (10), l'alésage (18) étant positionné de manière non excentrique, c'est-à-dire non centré, sur la face plate de la tête (10) afin de recevoir l'organe de raccordement (12) et fournir un premier degré d'excentricité ;
une pièce de verrouillage (14) destinée à verrouiller l'organe de raccordement (12) de manière non rotative à l'intérieur de l'alésage (18) ;
la seconde partie (16) étant une pièce de raccordement mâle, d'un diamètre sensiblement inférieur à la première partie (15), qui s'étend de manière conique à partir de la première partie (15) et s'emboîte dans une cavité conique de la tige pour un emboîtement de cône Morse, **caractérisée en ce que** la seconde partie (16) s'étend selon un décalage axialement parallèle par rapport à la première partie (15) afin de fournir un second degré d'excentricité par la relation entre la seconde partie (16) et la première partie (15).

2. Prothèse selon la revendication 1, dans laquelle la première partie (15) de l'organe de raccordement (12) est configurée de façon à être insérée en rotation dans l'alésage (18) axial de la tête humérale (10), afin de permettre un ajustement de la seconde partie (16) de l'organe de raccordement (12) par rapport à l'axe de la partie sphérique.

3. Prothèse selon la revendication 1, dans laquelle la seconde partie (16) de l'organe de raccordement (12) est une broche faisant saillie comportant un cône Morse.

4. Prothèse selon la revendication 1, dans laquelle l'alésage de la tête humérale (10) et la pièce de verrouillage (14) sont filetés de façon à entrer en prise.

5. Prothèse selon la revendication 1, dans laquelle la pièce de verrouillage (14) est une bague de verrouillage comportant un alésage ménagé à travers celle-ci avec une surface interne et un épaulement périphérique mettant en prise la première partie (15) de l'organe de raccordement (12).

6. Prothèse selon la revendication 5, dans laquelle au moins une rainure est pratiquée le long de la surface interne de la bague afin de recevoir un outil destiné à ajuster la bague.

7. Prothèse selon la revendication 1, dans laquelle la pièce de verrouillage (14) comporte une surface de mise en prise avec un outil afin de recevoir un outil destiné à ajuster la pièce de verrouillage (14).

8. Prothèse selon la revendication 1, comprenant en outre un cliquet formé sur la première partie (15) de l'organe de raccordement (12) et une série d'évidements formés à l'intérieur de l'alésage (18) axial de la tête humérale (10) destinés à entrer en prise avec le cliquet afin de permettre à l'organe de raccordement (12) d'être ajusté de manière rotative par rapport à la tête humérale (10) dans des positions prédéterminées.
